# FASCICULE DE BREVET EUROPEEN

(11) **EP 2 707 378 B1**
(45) Date de publication et mention de la délivrance du brevet: **02.08.2017**
(21) Numéro de dépôt: 12725131.2
(22) Date de dépôt: 10.05.2012
(51) Int. Cl.: C07D 249/06, H01G 5/18, H01G 11/02, H01G 11/26, H01G 11/58, H01G 11/60, H01G 11/62, H01M 10/05, H01M 10/0567, C07F 17/02, C09K 9/02, C07D 333/34, H01M 10/052

(54) **COMPOSÉS À GROUPEMENT REDOX, LEUR UTILISATION COMME ADDITIF D'ÉLECTROLYTE, COMPOSITION D'ÉLECTROLYTE ET SYSTÈMES ÉLECTROCHIMIQUES LES CONTENANT.**
VERBINDUNGEN MIT EINER REDOXGRUPPE, IHRE VERWENDUNG ALS ELEKTROLYTZUSATZ, ELEKTROLYTZUSAMMENSETZUNG UND ELEKTROCHEMISCHE SYSTEME DAMIT
COMPOUNDS HAVING A REDOX GROUP, USE THEREOF AS AN ELECTROLYTE ADDITIVE, ELECTROLYTE COMPOSITION, AND ELECTROCHEMICAL SYSTEMS CONTAINING SAME

(30) Priorité: 12.05.2011 FR 1154137
(43) Date de publication de la demande: 19.03.2014
(73) Titulaire: Centre National de la Recherche Scientifique (C.N.R.S.), 75016 Paris (FR); Université de Nantes, 44000 Nantes (FR)
(72) Inventeur: GAUBICHER, Joël, F-44000 Nantes (FR); MADEC, Lénaïc, F-44000 Nantes (FR); BOUVREE, Audrey, F-49270 Saint Laurent des Autels (FR); BLANCHARD, Philippe, F-49070 Saint Lambert La Potherie (FR); LESTRIEZ, Bernard, F-44000 Nantes (FR); BROUSSE, Thierry, F-44240 La Chapelle Sur Erdre (FR); GUYOMARD, Dominique, F-44880 Sautron (FR)
(74) Mandataire: Goulard, Sophie
(86) Numéro de dépôt international: PCT/FR2012/051032
(87) Numéro de publication internationale: WO 2012/153067

(56) Documents cités:
- GB-A- 2 467 148
- KLARE, J.E. ET AL.: "Assembling p-type molecules on single wall carbon nanotubes for photovaltaic devices", CHEMICAL COMMUNICATIONS, 2009, pages 3705-3707, XP002665334,
- LE GOFF, A. ET AL.: "Facile and tunable funtionalization of carbon nanotube electrodes with ferrocene by covalent coupling and pi-stacking interactions and their relevance to glucoe bio-sensing", JOURNAL OF ELECTROANALYTICAL CHEMISTRY, vol. 641, no. 1-2, 2010, pages 57-63, XP002665335,

## Description

La présente invention est relative à des composés comportant un groupement redox, à leur utilisation à titre d'additif dans une composition d'électrolyte, à une composition d'électrolyte comprenant un tel additif, et aux systèmes électrochimiques comprenant une telle composition d'électrolyte, notamment les accumulateurs au lithium ou au sodium (ou batteries au lithium ou au sodium) et les supercondensateurs à couche double électrique.

De manière connue, les accumulateurs se composent d'une électrode positive (cathode), le plus souvent un oxyde de métal de transition (dioxyde de cobalt ou manganèse) et d'une électrode négative en graphite (anode) entre lesquelles est placé un séparateur imprégné d'un électrolyte constitué d'un sel de lithium ou de sodium en solution dans un solvant choisi pour optimiser le transport et la dissociation des ions (en général un mélange de carbonates). Un collecteur de courant est relié à la cathode pour assurer la connexion électrique.

Les systèmes électrochimiques à stockage à couche double électrique et/ou pseudo capacitifs tels que les supercondensateurs sont des dispositifs de stockage d'énergie dont le principe de base repose sur les propriétés capacitives de l'interface entre un conducteur électronique solide et un conducteur ionique liquide. Un supercondensateur se compose généralement de deux collecteurs de courant métalliques, généralement en aluminium, de deux électrodes carbonées poreuses imprégnées d'électrolyte, et d'une membrane de séparation poreuse. Le stockage d'énergie s'effectue par distribution des ions de l'électrolyte au voisinage de la surface de chaque électrode sous l'influence électrostatique de la tension appliquée. Il se créé ainsi aux interfaces une zone de charge d'espace, appelée couche double électrique, d'épaisseur limitée à quelques nanomètres, et dans laquelle il règne un champ électrique relativement intense (de l'ordre de 10 kV.µm⁻¹).

Les systèmes électrochimiques à électrodes faradiques tels que les batteries au lithium, présentent de bonnes performances en termes d'énergie mais sont peu efficaces en termes de puissance. C'est l'inverse pour les systèmes électrochimiques à stockage à couche double et/ou pseudo-capacitif tels que les supercondensateurs par exemple. De plus les systèmes électrochimiques à électrodes faradiques telles que les batteries au lithium ou les supercondensateurs hybrides par exemple, fonctionnent tous grâce à une électrode dans laquelle la matière active d'électrode doit être préalablement (co-) mélangée avec un ou des polymères ou déposée avec/sur le ou les agents conducteurs des électrodes avant assemblage du système électrochimique, de façon à obtenir des contacts électriques efficaces, condition *sine qua non* des bonnes performances de ces systèmes. Il s'agit de l'étape de mise en forme de l'électrode.

Différentes méthodes ont déjà été envisagées pour améliorer les performances, notamment la puissance, de ces systèmes électrochimiques.

Certains auteurs, tels que Qin Y. et al. (Journal of Power Sources, 2010, 195, 6888-6892) ont testé l'ajout de 3,9-divinyl-2,4,8,10-tetraoxaspiro-[5,5] undécane (TOS) dans un électrolyte à base pour augmenter la durée de vie et la stabilité thermique des batteries au lithium comportant une électrode négative à base de microbilles de mésocarbone (MCMB), de fibres de carbone et de polyfluorure de vinylidène (PVDF) dispersées dans la N-méthyl-2-pyrrolidone sur une feuille de cuivre, une électrode positive à base de Li[Ni_{1/3}Co_{1/3}Mn_{1/3}]O₂ (NCB), de noir de carbone et de PVDF dispersés dans la N-méthyl-2-pyrrolidone sur une feuille d'aluminium, un séparateur en polyprolylène microporeux et LiPF₆ à titre d'électrolyte. Les auteurs indiquent que l'ajout de TOS à raison de 1 % massique dans l'électrolyte améliore la capacité de rétention de la batterie (en fonctionnement à une température de 55°C) et sa stabilité thermique, mais qu'une quantité supérieure à 0,5% de ce même composé provoque par contre une chute importante de l'impédance.

Yu H. et al. (Electrochemical and Solid-State Letters, 2004, 7(11), A442-A446) ont testé l'emploi de carbonate d'éthylène de vinyle à titre d'additif dans un électrolyte à base de carbonate de propylène et de lithium bis(perfluoroéthylsulfonyl) imide (LiBETI) dans des batteries au lithium comportant soit une anode préparée à partir d'une poudre de graphite soit une anode préparée à partir de MCMB. La présence de cet additif dans l'électrolyte permet de créer un film protecteur à la surface de l'électrode de façon à empêcher les ions lithium de s'insérer dans le graphite et de le détériorer, ce qui a pour conséquence d'améliorer les performances de la batterie. Cependant les auteurs indiquent que cet effet n'est observé qu'avec la batterie comportant l'électrode préparée à partir de la poudre de graphite mais pas à celle dans laquelle l'électrode a été préparée à partir de MCMB. Cette méthode n'est donc pas applicable à tout type d'électrode carbonée, encore moins à tout type d'électrode.

D'autres méthodes mettent en oeuvre des navettes redox, c'est-à-dire des batteries dans lesquelles l'électrolyte contient, à titre d'additif un composé redox ou un polymère réactif qui, lors du fonctionnement de la batterie, fait la navette entre les deux électrodes (Z Chen et al., Electrochimica Acta, 2009, 54, 5605-5613), dans le but d'éviter les surcharges. De tels composés redox peuvent par exemple être des composés aromatiques tels que le 1,4-di-terbutyl-2,5-diméthoxybenzène, le 2,2,6,6-tétraméthylpipéridinyl-1-oxyle (TEMPO) ou encore le 2-(pentafluorophényl)-tetrafluoro-1,3,2-benzodioxaborole (PFPTFBB), des composés non aromatiques tels que certains sels de lithium boratés comme Li₂B₁₂F₁₂₋ₓHₓ avec x = 1-12. Les auteurs indiquent cependant qu'il reste difficile de trouver des composés qui ne finissent pas par se décomposer et qui restent efficaces au cours du temps. Par ailleurs, ces composés doivent diffuser dans l'électrolyte pour assurer une réversibilité redox. Ils ne sont donc pas localisés à une électrode, ce qui les rend incapables de délivrer et/ou d'emmagasiner de l'énergie à haute puissance.

Enfin, d'autres méthodes prévoient la fonctionnalisation des électrodes de carbone par des groupements organiques de type carboxylique ou amino tel que décrit par exemple par Lee et al. (Nature Nanotechnology, 2010, 5, 531-537), des groupements sulfophényle tel que décrit par D. Pech et al. (Electrochemical and Solid-State Letters, 2008, 11, A202-A205) ou encore par des groupements aromatiques, par exemple de type pyrène, tel que décrit notamment par C. Ehli et al. (JACS, 2006, 128, 11222-11231). Ces méthodes nécessitent cependant de procéder à la fonctionnalisation moléculaire du matériau actif d'électrode (graphite ou carbone) avant l'assemblage des électrodes non classiques, ce qui nécessite donc une étape supplémentaire.

Les inventeurs se sont donc donné pour but de proposer une solution qui permette d'améliorer la puissance des systèmes électrochimiques à électrodes faradiques, ainsi que l'énergie des systèmes à électrodes à couche double électrique et/ou pseudo-capacitives, qui soit facile, rapide et simple à mettre en oeuvre. En particulier, les inventeurs se sont fixé pour but de proposer une solution qui évite toute étape supplémentaire de mise en forme de l'électrode ou le recours à un système hybride contenant une électrode de batterie et une électrode de supercondensateur.

Les inventeurs ont mis au point de nouveaux composés comportant un groupement redox et dont l'utilisation, à titre d'additif dans une composition d'électrolyte, permet de remédier aux inconvénients des systèmes de l'art antérieur, et en particulier permet d'améliorer la puissance des systèmes électrochimiques à électrodes faradiques, ainsi que l'énergie des systèmes à électrodes à couche double électrique et/ou pseudo-capacitives. On préfère les composés de formules (1-1) à (1-3) suivantes :

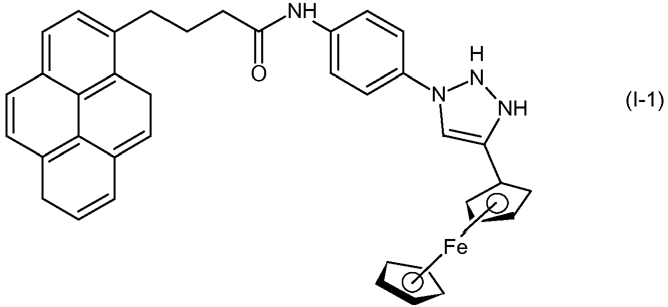

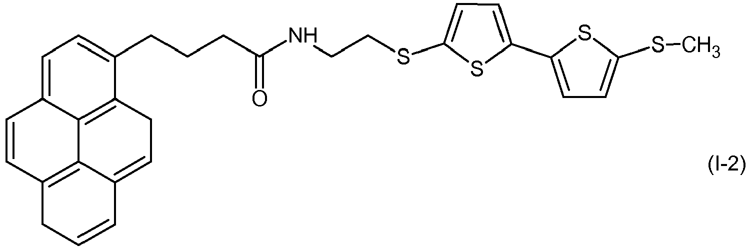

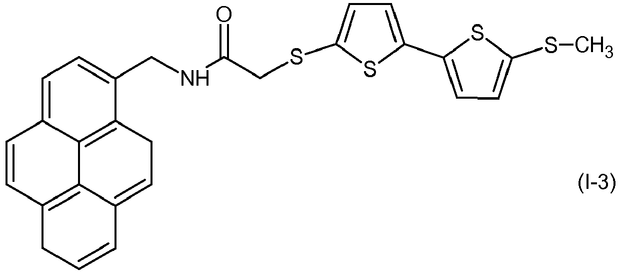

Les composés de formule (I) (I = I-1, I-2, I-3) conformes à l'invention peuvent être préparés par des méthodes de synthèse connues de l'homme du métier, par condensation d'un précurseur d'un groupement A avec un précurseur d'un groupement Redox.

Un autre objet de l'invention est l'utilisation d'au moins un composé de formule (I) tel que défini ci-dessus, à titre d'additif dans une composition d'électrolyte d'un système électrochimique à électrodes faradiques et/ou à électrodes à couche double électrique ou pseudo-capacitives fonctionnant par circulation d'ions entre une électrode positive et une électrode négative séparées par une composition d'électrolyte.

L'invention a donc également pour objet une composition d'électrolyte pour un système électrochimique à électrodes faradiques et/ou à électrodes à couche double électrique ou pseudo-capacitives fonctionnant par circulation d'ions entre une électrode positive et une électrode négative séparées par une composition d'électrolyte, ladite composition d'électrolyte étant caractérisée en ce qu'elle renferme :
- au moins un composé ionique,
- au moins un solvant liquide, et
- au moins un composé de formule (I) tel que défini ci-dessus.

La quantité de composé de formule (I) au sein de la composition d'électrolyte conforme à l'invention varie de préférence de 10⁻⁶ mol/l à 2.10⁻¹ mol/l, et encore plus préférentiellement de 10⁻⁴ mol/l à 10⁻¹ mol/l.

Selon une forme de réalisation préférée de l'invention, ladite composition d'électrolyte renferme au moins un composé de formule (1-1) tel que défini précédemment.

Selon une forme de réalisation particulière de l'invention, le système électrochimique est une batterie fonctionnant par circulation d'ions lithium ou sodium. Dans ce cas, l'électrolyte comprend généralement un sel de lithium ou de sodium en solution dans un solvant aprotique polaire choisi pour optimiser le transport et la dissociation des ions.

De tels sels peuvent être choisis notamment parmi les sels de lithium ou de sodium d'un anion répondant à l'une des formules suivantes : ClO₄⁻, BF₄⁻, PF₆⁻, AsF₆⁻, SbF₆⁻, SCN⁻, R_{F}SO₃⁻, [(R_{F}SO₂)NSO₂R'_{F}]⁻, [(R_{F}SO₂)C(Y)SO₂R_{F}']⁻ dans laquelle Y est CN ou SO₂R_{F}", [R_{F}SO₂(NCN)]⁻, [R_{F}SO₂{C(CN)₂}]⁻, 2-perfluoroalkyl-4,5-dicyanoimidazole [R_{F}C₅N₄]⁻, 4,5-dicyano-1,2,3-triazole [C₄N₅]⁻, 2,5-bis(fluorosulfonyl)-1,3,4-triazole [C₂F₂S₂O₄]⁻, et 3-cyano-5-perfluoroalkyle-1,3,4-triazole [R_{F}C₃N₄]⁻, R_{F}, R_{F}' et R_{F}" étant des groupes alkyle dans lesquelles au moins 60 % des atomes d'hydrogène sont remplacés par des atomes de fluor.

Selon une forme de réalisation préférée de l'invention, l'électrolyte comprend un sel de lithium choisi parmi LiPF₆, LiAsF₆, LiClO₄, LiBF₄, LiC₄BO₈, Li(C₂F₅SO₂)₂N, Lᵢ[(C₂F₅)₃PF₃], LiCF₃SO₃, LiCH₃SO₃, LiN(SO₂CF₃)₂, et LiN(SO₂F)₂.

Les composés polaires aprotiques du solvant liquide de la composition d'électrolyte sont de préférence choisis parmi les carbonates linéaires ou cycliques, les éthers linéaires ou cycliques, les esters linéaires ou cycliques, les sulfones linéaires ou cycliques, les sulfamides et les nitriles. Le solvant est constitué de préférence par au moins deux carbonates choisis parmi le carbonate d'éthylène, le carbonate de propylène, le carbonate de diméthyle, le carbonate de diéthyle et le carbonate de méthyle et d'éthyle.

Selon une autre forme de réalisation particulière de l'invention, le système électrochimique est un supercondensateur. Dans ce cas, la composition d'électrolyte peut être choisie parmi :
- les compositions d'électrolyte comprenant un sel de lithium ou de sodium en solution dans un solvant aprotique polaire et telles que décrites ci-dessus pour les batteries ;
- les solutions aqueuses de pH neutre (pH entre 5 et 9 environ) telles que les solutions de K₂SO₄ ou d'un sel de type chlorate ;
- les solutions aqueuses de pH acide (pH < 5), telles que les solutions d'acide sulfurique ;
- les solutions aqueuses de pH basique (pH > 9), telles que les solutions de potasse ou de soude.

Enfin, l'invention a pour objet un système électrochimique à électrodes faradiques et/ou à électrodes à couche double électrique ou pseudo-capacitives fonctionnant par circulation d'ions entre une électrode positive et une électrode négative séparées par une composition d'électrolyte, caractérisé en ce que la composition d'électrolyte est telle que définie précédemment, et en ce que l'une et/ou l'autre des électrodes comprend au moins un matériau comportant des fonctions hydroxyle et/ou un matériau carboné et/ou un additif carboné.

Selon l'invention, ledit système peut notamment être une batterie au lithium, une batterie au sodium, un supercondensateur à couche double électrique, un système hybride comprenant une électrode de batterie (électrode faradique) et une électrode de supercondensateur (électrode à couche double électrique ou pseudocapacitive), ou bien encore une cellule électrochimique à flux continu (« *flow cell* »).

Lorsque que le système est une batterie ou un système hybride comportant une électrode positive de batterie (électrode faradique), ladite électrode positive est généralement constituée par un collecteur de courant portant une matière active d'électrode positive, éventuellement un agent de conduction électronique, et éventuellement un liant. La teneur en matière active dudit matériau composite est de préférence de 5 à 95% en poids, la teneur en agent de conduction électronique est de préférence de 0,1 à 30% en poids, la teneur en liant est de préférence inférieure à 25% en poids.

Dans, ce cas, et lorsque le dispositif électrochimique conforme à l'invention fonctionne par circulation d'ions lithium, le matériau actif de l'électrode positive est de préférence choisi parmi :
- les oxydes de métaux de transition à structure spinelle de type LiM₂O₄ et les oxydes de métaux de transition à structure lamellaire de type LiMO₂ dans lequel M représente au moins un métal choisi dans le groupe constitué par Mn, Fe, Co, Ni, Cu, Mg, Zn, V, Ca, Sr, Ba, Ti, Al, Si, B et Mo ;
- les oxydes à charpente polyanionique de type LiM_{y}(XO_{z})ₙ dans lesquels M représente au moins un métal choisi dans le groupe formé par Mn, Fe, Co, Ni, Cu, Mg, Zn, V, Ca, Sr, Ba, Ti, Al, Si, B et Mo et X représente un élément choisi dans le groupe constitué par P, Si, Ge, S et As ;
- les oxydes à base de vanadium de type V₂O₅, LiV₃O₈ et leurs dérivés ;
- les oxydes à base de manganèse de type MnO₂ et ses dérivés ;
- le soufre, le phosphore.

Parmi les oxydes à structure spinelle de type LiM₂O₄, on préfère ceux dans lesquels M représente au moins un métal choisi parmi par Mn et Ni. Parmi les oxydes à structure lamellaire de type LiMO₂, on préfère ceux dans lesquels M représente au moins un métal choisi parmi par Mn, Co et Ni. Parmi les oxydes à charpente polyanionique de type LiM_{y}(XO_{z})_{w}, on préfère en particulier les phosphates à structures olivine, dont la composition répond à la formule LiMPO₄ dans laquelle M représente au moins un élément choisi parmi Mn, Fe, Co et Ni. LiFePO₄ est préféré.

Toujours dans ce cas, et lorsque le dispositif électrochimique conforme à l'invention fonctionne par circulation d'ions sodium, le matériau actif de l'électrode positive est de préférence choisi parmi :
- les fluorophosphates lamellaires Na₂TPO₄F dans lesquels T représente un élément divalent choisi parmi Fe, Mn, Co, et Ni, qui peuvent être remplacés partiellement par Mg ou Zn, ou un ion divalent VO ;
- les fluorosulfates NaT'SO₄F dans lesquels T' représente au moins un élément choisi parmi Fe, Mn, Co et Ni, dont une partie peut être remplacée par Mg et une partie des groupements sulfates SO₄²⁻ peut être remplacés par le groupement iso-stère et iso-charge PO₃F²⁻ ;
- les polysulfures Na₂Sₙ (1≤n≤6), et les sels de sodium du dimercaptothiadiazole et du dimercaptooxazole ;
- les dithiocarbamates Na[CS₂NR'R"] dans lesquels chacun des groupes R' et R" représente un radical méthyle, éthyle, ou propyle, ou bien R' et R" formant un cycle (par exemple la pyrolidine ou la morpholine).

Parmi de tels matériaux actifs d'électrode positive, Na₂FePO₄Fe et NaFeSO₄F sont préférés.

L'agent de conduction électronique de l'électrode positive est un agent carboné pouvant notamment être choisi parmi le graphite, les noirs de carbone graphitique, le graphène, les nanofibres de carbone, les nanotubes de carbone.

Comme indiqué précédemment, l'électrode positive peut en outre comprendre un liant, en particulier un liant polymère destiné à assurer la cohésion entre les particules du matériau actif d'électrode et l'agent de conduction électronique. Parmi de tels liants polymères, on peut notamment citer les polysaccharides, les polysaccharides modifiés, les latex, les polyélectrolytes, les polyéthers, les polyesters, les polymères polyacryliques, les polyuréthanes, les polyépoxydes, les polyphosphazènes, les polysulfones, les polymères halogénés.

Parmi de tels polymères, on peut plus particulièrement citer le polytétrafluoroéthylène (PTFE), la carboxyméthylcellulose (CMC), le polyfluorure de vinylidène (PVDF).

Lorsque que le système est une batterie ou un système hybride comportant une électrode négative de batterie (électrode faradique), ladite électrode négative peut en particulier être constituée de lithium métallique (de préférence sous forme de film), un alliage de lithium, de graphite, de fibres de carbone, de nanotubes de carbone, de noir d'acétylène ou bien encore d'un collecteur de courant portant une matière composite d'électrode, comprenant une matière active d'électrode négative telle que le silicium, l'étain, les oxydes de titanes, les oxydes de Co, de Cu, etc ..., éventuellement un agent de conduction électronique choisi parmi ceux définis précédemment pour l'électrode positive, et éventuellement un liant.

Lorsque que le système est un supercondensateur à couche double électrique ou pseudocapacitive, les électrodes positive et négative sont généralement constituées d'un matériau carboné poreux choisi parmi le graphite, les noirs de carbone graphitique, le graphène, les nanofibres de carbone, les nanotubes de carbone, ledit matériau poreux étant imprégné de la composition d'électrolyte. Lesdites électrodes peuvent en outre comprendre un matériau actif d'électrode choisis parmi certains oxydes tels les oxydes de manganèse ou de ruthénium, et les nitrures.

Dans les systèmes hybrides, les électrodes positive et négative peuvent être choisies respectivement parmi les électrodes décrites ci-dessus pour les systèmes à électrodes faradiques et les supercondensateurs.

### EXEMPLES

### Exemple 1 : Synthèse du composé de formule (I-1)

La synthèse du composé de formule (1-1) a été réalisée selon le schéma de synthèse suivant :

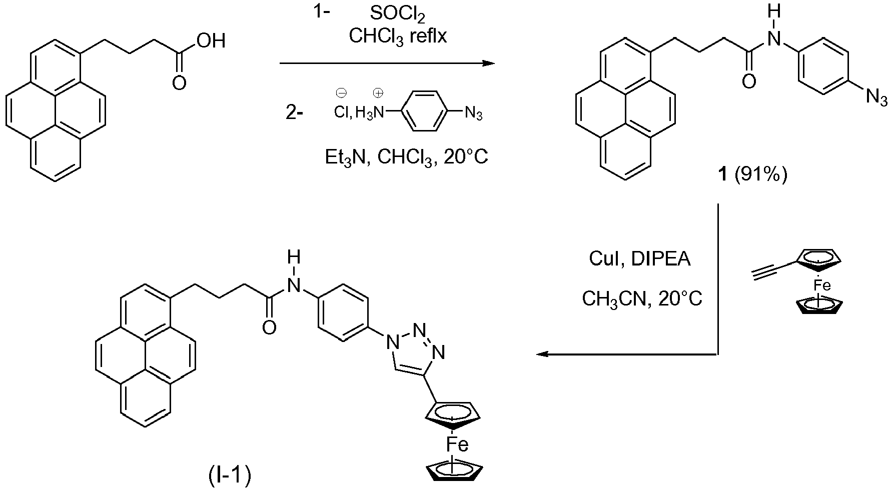

Les spectres RMN ont été enregistrés sur un appareil Bruker AVANCE III 300 (¹H, 300 MHz et ¹³C, 75 MHz).

Les déplacements chimiques sont exprimés en parties par million (ppm) par rapport au TMS.

Les spectres infrarouges ont été enregistrés sur un spectromètre Bruker Vertex 70.

La désorption-ionisation laser assistée par matrice a été réalisée sur un spectromètre MALDI-TOF MS BIFLEX III Bruker Daltonics avec le dithranol pour matrice.

Les spectres de masse par ionisation par électronébulisation (électrospray) (ESI) ont été enregistrés à l'aide d'un spectromètre JMS Jeol 700.

### 1) Première étape : Synthèse du composé 1

Sous atmosphère d'azote, le chlorure de thionyle (0,8 mL, 11 mmol) a été lentement ajouté, à l'aide d'une seringue, dans une solution d'acide 4-(pyrèn-1-yl)butanoïque (2 g, 6,94 mmol) dans CHCl₃ anhydre (20 mL), placée dans un tricol de 50 mL. Le mélange réactionnel a été maintenu 2 h à reflux, puis refroidi à température ambiante et concentré sous vide. 20 mL de CH₂Cl₂ ont alors été ajoutés au mélange et le solvant a été évaporé sous vide afin d'éliminer l'excès de chlorure de thionyle. Cette procédure a été répétée deux fois et a permis d'obtenir le chlorure d'acyle intermédiaire.

Sous atmosphère d'azote, une solution de cet intermédiaire dans CHCl₃ anhydre (20 mL) a été ajoutée goutte à goutte dans une solution d'hydrochlorure de 4-azidoaniline (1,2 g, 7,03 mmol) dans CHCl₃ anhydre (20 mL) et en présence de triéthylamine (2 mL, 14,4 mmol). Le mélange réactionnel a été agité pendant 15 h, conduisant à la formation d'un précipité blanc gris. Le solvant a été évaporé et le produit brut a été extrait avec de l'acétate d'éthyle (EtOAc) chaud (3 x 250 mL). Les extraits ont été rassemblés et lavés avec une solution aqueuse de NaCl saturée (2 x 150 mL), séchés sur MgSO₄ et concentrés pour donner le composé **1** sous forme de poudre beige (2.54 g, rendement 91%).
Température de fusion : 176-177°C.
RMN ¹H (300 MHz, DMSO-d6) *δ*: 9.99 (br. s, 1H, N-H) ; 8.41 (d, 1H, ³J=9.3Hz) ; 8.29-8.20 (m, 4H) ; 8.15 (d, 1H, ³J=9.1Hz) ; 8.12 (d, 1H, ³J=9.1Hz) ; 8.06 (t, 1H, ³J=7.7Hz) ; 7.97 (d, 1H, ³J=7.7Hz) ; 7.65 (d, 2H, ³J=8.7Hz) ; 7.06 (d, 2H, ³J=8.7Hz) ; 3.38 (t, 2H, ³J=7.5Hz); 2.46 (t, 2H, ³J=7.2Hz); 2.11 (quint., 2H, ³J=7.5Hz).
IR: ṽ= 3253 cm⁻¹ (N-H), 2116 cm⁻¹ (-N₃), 1655 cm⁻¹ (C=O).
ESI⁻ MS: 439.4 [M+Cl⁻], 842.8 [2M+Cl⁻].

### 2) Deuxième étape : synthèse du composé de formule (I-1)

107,8 µL de *N,N-*Diisopropyléthylamine (DIPEA ; 0,65 mmol) et 7,3 mg de CuI (0,04 mmol) ont été ajoutés à une solution du composé **1** obtenu ci-dessus à l'étape précédente (100 mg, 0,25 mmol) et de 52 mg d'éthynylferrocène (0,25 mmol) dans CH₃CN (7,5 mL). Le mélange a été agité 5 jours à température ambiante. Après dilution dans EtOAc (100 mL), la phase organique a été lavée avec une solution aqueuse de NaCl saturée (2 x 20 mL), séchée sur MgSO₄ et concentrée sous vide permettant d'obtenir une poudre légèrement marron.
Température de fusion : 270-272°C.
RMN ¹H (300 MHz, pyridine-d5) *δ*: 11.01 (1. s, 1H, N-H) ; 8.44 (d, 1H, ³J=9.3Hz) ; 8.26-7.94 (m, 12H) ; 7.87 (d, 1H, ³J=7.8Hz) ; 5.03 (1. s, 2H, Fc) ; 4.35 1. s, 2H, Fc) ; 4.16 (1 s, 5H, Fc) ; 3.47 (t, 2H, ³J=7.5Hz) ; 2.63 (t, 2H, ³J=7.2Hz) ; 2.40 (quint., 2H, ³J=7.2Hz).
IR : ṽ= 3347 cm⁻¹ (N-H), 1670 cm⁻¹ (C=O).
MALDI-TOF MS : 613.8 [M⁺].

### Exemple 2 : Préparation d'une cellule électrochimique

Dans cet exemple, on a préparé une cellule électrochimique comprenant une électrode de travail (électrode positive) comprenant des nanotubes de carbone, une contre électrode (électrode négative) constituée d'une feuille de lithium métallique laminée sur un collecteur de courant en inox et d'un fritté à titre de séparateur, l'ensemble étant plongé dans un électrolyte liquide.

L'électrode de travail (3 mg) a été préparée par mélange manuel de nanotubes de carbone à paroi multiple défagotés (MWCNT : « *Multi Walled Carbon NanoTubes* ») vendus sous la dénomination commerciale MWNT (95+%, OD 20-30 nm) par la société Nanostructured & Amorphous Materials Inc. (NanoAmor), selon le procédé décrit dans la demande de brevet FR 2 935 546, et de polytetrafluoroéthylène vendu sous la dénomination commerciale Teflon ® par la société Aldrich, dans un rapport massique respectif de 95/5. Le mélange obtenu a été pressé à 10 Tonnes/cm² sur une grille d'inox.

Ces deux électrodes ont été placées dans un électrolyte constitué d'une solution 1M de LiPF₆ dissous dans un mélange de carbonate d'éthylène (EC) et de diméthylcarbonate (DMC) (1/1 (v/v)) et sont de part et d'autre d'une électrode de référence constituée par du lithium métal plongée dans le même électrolyte et séparée par un fritté.

Le système a été cyclé 5 fois à 50 mV/s (88 s/cycle) pour tester la connectique avant de dissoudre dans l'électrolyte, 0,625 mg du composé redox de formule (1-1) tel que synthétisé ci-dessus à l'exemple 1, pour 5 ml d'électrolyte :
Après ajout du composé de formule (1-1), le système a été mis à cycler dans les mêmes conditions.

La capacité de l'électrode de travail a été déterminée avant et après ajout du composé de formule (1-1) par coulométrie.

Avant ajout du composé de formule (1-1), la capacité de l'électrode de travail était d'environ 0,015 mAh (5 mAh/g). Cette capacité correspond à la capacité de double couche de l'agent conducteur (7 F/g). On en déduit que la surface électrochimiquement active de l'électrode est d'environ 87 m²/g en prenant comme référence 10 µF/g pour la capacité de double couche. Cette valeur est en bon accord avec la surface BET des nanotubes de carbone initiaux qui est de l'ordre de 110 m²/g.

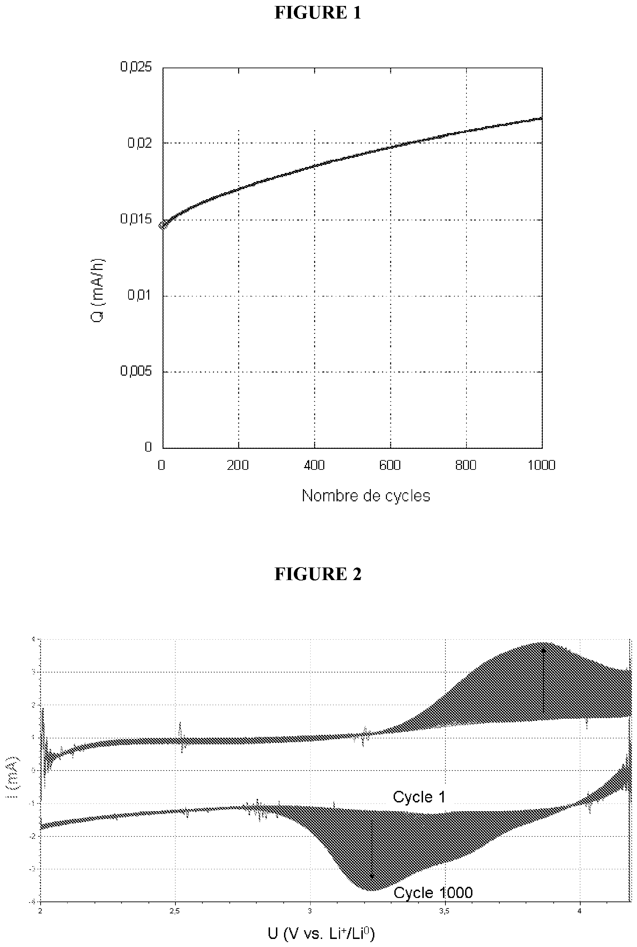

L'évolution de la capacité de l'électrode a été suivie pendant au moins 24 heures et est reportée sur la figure 1 annexée sur laquelle la décharge Q (mA.h) est exprimée fonction du nombre de cycles, le cercle sur la courbe représentant le moment où à été ajouté le composé de formule (I-1) dans l'électrolyte.

Sur cette figure on constate que dès l'ajout du composé de formule (1-1), la capacité de l'électrode a augmenté pour atteindre 0,022 mA.h après 1000 cycles soit environ 24 heures.

Cette augmentation de capacité intervient suite à l'activation faradique de l'électrode qui se traduit par la croissance d'une vague redox à environ 3,25 V en décharge et 3,85 V en charge (voir la figure 2 annexée sur laquelle l'intensité du courant I (mA) est fonction du potentiel (en volts) vs Li⁺/Li⁰.

Ce signal indique que le composé de formule (1-1) se fixe de façon pérenne sur l'agent conducteur, ici les nanotubes de carbone, permettant ainsi de stocker/délivrer environ 1,5 fois plus d'énergie et de puissance qu'initialement (16 Wh/kg_{electrode}, 1,3 kW/kg_{electrode} contre 23 Wh/kg_{electrode}, 1,9 kW/kg_{electrode}, correction faite de la masse de molécule greffée.

Ce résultat démontre que l'activation faradique de l'électrode peut être effectuée pendant le cyclage. La quantité de composé de formule (1-1) greffé est de l'ordre de 1,5 .10⁻¹¹ mol/cm² ce qui correspond à une vitesse de greffage moyenne de l'ordre de 0,06.10⁻¹¹ mol/cm²/heure. On note que le greffage du composé de formule (I-1) n'altère pas l'amplitude la capacité de double couche.

### Exemple 3

Une électrode de travail de 1 mg, identique par ailleurs en tout point à celle préparée ci-dessus à l'exemple 2 a été immergée pendant 120 heures dans une solution du composé de formule (1-1) à 2.10⁻⁴mol/l dans la solution électrolytique initiale.

Après immersion, l'électrode a été cyclée selon le même montage et dans les mêmes conditions que celles exposées ci-dessus à l'exemple 2. Dans ce cas, la capacité initiale de l'électrode a atteint 0,011 mAh à 100 mV/s (44 s/cycle) alors qu'elle n'était que de 0,006 mAh initialement, permettant ainsi de stocker/délivrer 2 fois plus d'énergie et de puissance qu'initialement (19 Wh/kg_{electrode}, 3 kW/kg_{electrode} contre 35 Wh/kg_{electrode}, 6 kW/kg_{electrode}) pendant au moins 1000 cycles.

Ce résultat démontre que l'activation faradique de l'électrode peut être effectuée avant le cyclage. La quantité de composé de formule (1-1) fixé était de l'ordre de 6,8 10⁻¹¹ mol/cm² ce qui est inférieur à la valeur attendue (7,2 mol/cm²) compte tenu de la quantité dissoute et pour une vitesse de greffage déterminée à 0,06 10⁻¹¹ mol/cm²/heure dans l'exemple 2. Ce résultat indique que la saturation de la surface est atteinte. La seule limitation à l'augmentation des performances obtenues en utilisant un composé de formule (I) à titre d'additif dans un électrolyte apparaît donc être la surface développée par l'agent conducteur de l'électrode.

### Exemple 4

Dans cet exemple, on a préparé une cellule électrochimique identique à celle préparée ci-dessus à l'exemple 2, mais en substituant les nanotubes de carbone par des fibres de carbone vendues sous la dénomination commerciale CFx par la société Showa Denko K.K., dans une électrode de 0,95 mg.

L'électrode a ensuite été testée en cyclage comme décrit ci-dessus à l'exemple 2, avant et après ajout du composé de formule (1-1).

Avant ajout du composé de formule (1-1), la capacité de l'électrode était d'environ 0,012 mAh (13 mAh/g), cette capacité correspondant à la capacité de double couche de l'agent conducteur (19 F/g). On en a déduit que la surface électrochimiquement active de l'électrode était d'environ 190 m²/g en prenant comme référence 10 µF/g pour la capacité de double couche.

Cette valeur était en bon accord avec la surface BET des fibres de carbone initiales qui est de l'ordre de 245 m²/g.

Dès l'ajout du composé de formule (1-1), la capacité a augmenté pour atteindre 0,033 mAh (30 mAh/g) après 8600 cycles soit environ 115 heures.

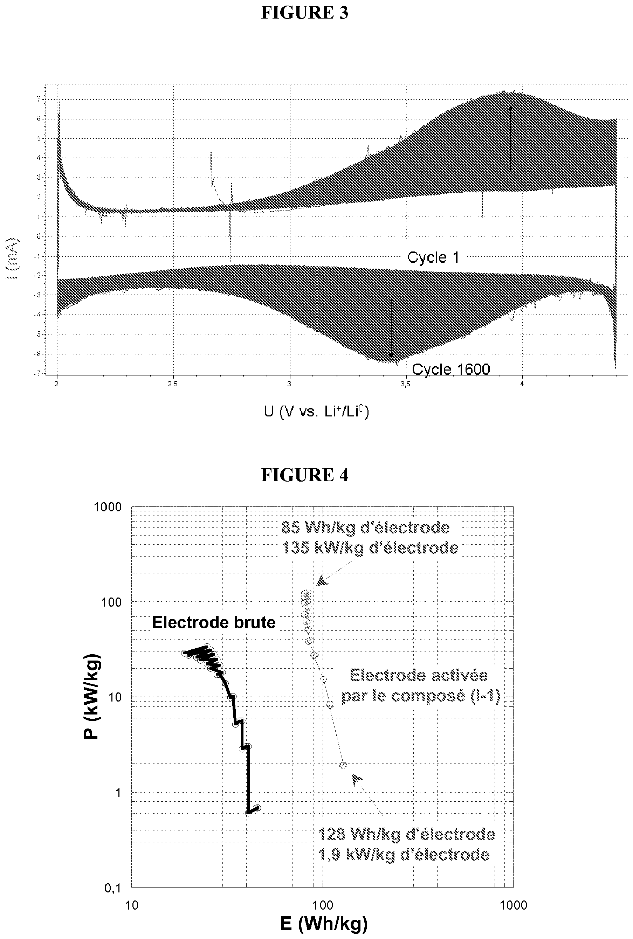

Cette augmentation de capacité intervient suite à l'activation faradique de l'électrode qui se traduit par la croissance d'une vague redox à environ 3,45 V (voir figure 3 annexée sur laquelle l'intensité du courant I (mA) est fonction du potentiel (en volts) vs Li⁺/Li⁰).

Ce signal indique que le composé de formule (1-1) se fixe de façon pérenne sur les fibres de carbone permettant ainsi de stocker/délivrer près de 2,5 fois plus d'énergie et de puissance massique qu'initialement (40 Wh/kg_{électrode}, 6 kW/kg_{électrode} contre 100 Wh/kg_{électrode}, 15 kW/kg_{électrode}), correction faite de la masse du composé de formule (1-1) greffé.

La quantité de composé de formule (1-1) greffé est de l'ordre de 8,6.10⁻¹¹ mol/cm², ce qui correspond à une vitesse de greffage moyenne de l'ordre de 0,075.10⁻¹¹ mol/cm²/heure ce qui est d'environ 20 % supérieure à celle observée ci-dessus à l'exemple 2 en mettant en oeuvre une électrode élaborée à partir de nanotubes de carbone. On note que le greffage du composé de formule (1-1) n'altère pas la capacité de double couche. Ce résultat démontre que les gains énergétiques et de puissance massique apportés par le composé de formule (1-1) sont proportionnels à la surface développée par l'agent conducteur de l'électrode et dépendent de sa nature, le meilleur rendement temps/performance étant obtenu avec les fibres de carbone.

### Exemple 5

Une électrode de travail analogue à celle utilisée ci-dessus dans l'exemple 4 a été cyclée pendant 3750 cycles dans les mêmes conditions que celle de l'exemple 4.

A titre de comparatif, une électrode en tout point identique a également été cyclée pendant 3750 cycles dans un électrolyte exempt de composé de formule (I-1).

Les résultats d'un test de puissance massique effectué sur chacune des électrodes est reporté sur la figure 4 annexée qui représente le diagramme de Ragone des électrodes testées. Sur cette figure, la puissance massique (P) exprimée en kW/kg est fonction de la densité de puissance (E) exprimée en Wh/kg. La courbe en trait noir épais correspondant à l'électrode témoin cyclée dans un électrolyte classique, c'est-à-dire ne contenant pas le composé de formule (1-1) et la courbe en trait noir fin correspond à l'électrode cyclée dans l'électrolyte conforme à l'invention, c'est-à-dire contenant le composé de formule (1-1)

Ces résultats montrent que lorsque l'on utilise un électrolyte conforme à l'invention, c'est-à-dire renfermant un composé de formule (1-1), une électrode de 1 mg est capable de délivrer 85 Wh/kg_{electrode} (0,425 mWh/m²_{electrode}) en 2,4 secondes, soit une puissance d'environ 130 kW/kg_{electrode} (0,65 mW/m²_{electrode}). La même électrode cyclée dans un électrolyte classique montre des performances environ 5 fois inférieures.

Ces performances constituent, à la connaissance des inventeurs, le meilleur compromis énergie-puissance jamais obtenu pour des électrodes dont la charge est importante (10 mg/cm²).

L'utilisation d'un électrolyte conforme à l'invention permet donc, en plus de tous les avantages précités, d'obtenir un objet capable de délivrer simultanément beaucoup d'énergie et de puissance, ce qui représente les avantages combinés des supercondensateurs et des batteries.

## Revendications

1. Composés de formules (1-1) à (1-3) suivantes :

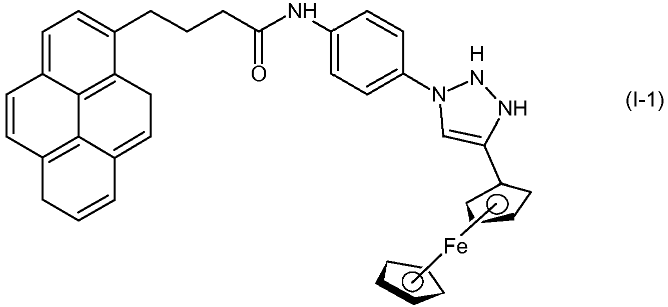

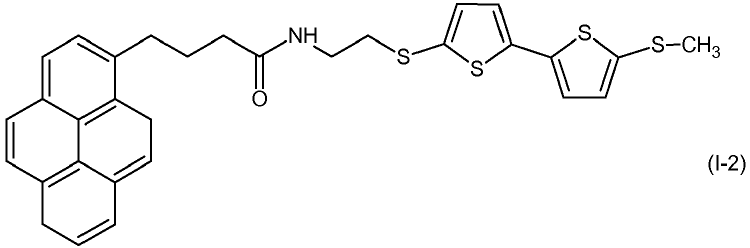

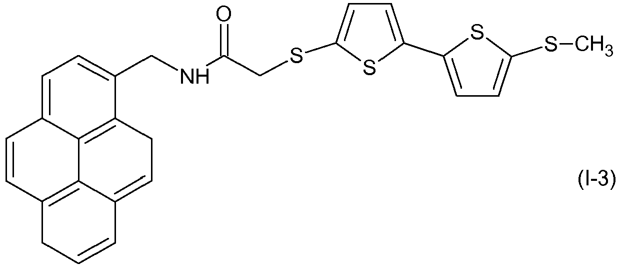

2. Utilisation d'au moins un composé choisi parmi les composés de formule (1-1) à (1-3) tels que définis à la revendication 1, à titre d'additif dans une composition d'électrolyte d'un système électrochimique à électrodes faradiques et/ou à électrodes à couche double électrique ou pseudo-capacitives fonctionnant par circulation d'ions entre une électrode positive et une électrode négative séparées par une composition d'électrolyte.

3. Composition d'électrolyte pour un système électrochimique à électrodes faradiques et/ou à électrodes à couche double électrique ou pseudo-capacitives fonctionnant par circulation d'ions entre une électrode positive et une électrode négative séparées par une composition d'électrolyte, ladite composition d'électrolyte étant **caractérisée en ce qu'**elle renferme :
- au moins un composé ionique,
- au moins un solvant liquide,
- au moins un composé choisi parmi les composés de formule (1-1) à (1-3) tels que définis à la revendication 1.

4. Composition selon la revendication 3, **caractérisée en ce que** la quantité de composé de formule (I-1), (1-2) et/ou (1-3) au sein de la composition d'électrolyte varie de 10⁻⁶ mol/l à 2.10⁻¹ mol/l.

5. Composition selon la revendication 3 ou 4, **caractérisée en ce qu'**elle renferme au moins un composé de formule (I-1) tel que défini à la revendication 1.

6. Composition selon l'une quelconque des revendications 3 à 5, **caractérisée en ce que** le système électrochimique est une batterie fonctionnant par circulation d'ions lithium ou sodium et **en ce que** l'électrolyte comprend un sel de lithium ou de sodium en solution dans un solvant aprotique polaire.

7. Composition selon la revendication 6, **caractérisée en ce que** l'électrolyte comprend un sel de lithium choisi parmi LiPF₆, LiAsF₆, LiClO₄, LiBF₄, LiC₄BO₈, Li(C₂F₅SO₂)₂N, Lᵢ[(C₂F₅)₃PF₃], LiCF₃SO₃, LiCH₃SO₃, LiN(SO₂CF₃)₂, et LiN(SO₂F)₂.

8. Composition selon l'une quelconque des revendications 3 à 5, **caractérisée en ce que** le système électrochimique est un supercondensateur et **en ce que** la composition d'électrolyte est choisie parmi :
- les compositions d'électrolyte comprenant un sel de lithium ou de sodium en solution dans un solvant aprotique polaire et telles que définies à l'une quelconque des revendications 6 ou 7 ;
- les solutions aqueuses de pH neutre telles que les solutions de K₂SO₄ ou d'un sel de type chlorate ;
- les solutions aqueuses de pH acide, telles que les solutions d'acide sulfurique ;
- les solutions aqueuses de pH basique, telles que les solutions de potasse ou de soude.

9. Système électrochimique à électrodes faradiques et/ou à électrodes à couche double électrique ou pseudo-capacitives fonctionnant par circulation d'ions entre une électrode positive et une électrode négative séparées par une composition d'électrolyte, **caractérisé en ce que** la composition d'électrolyte est telle que définie à l'une quelconque des revendications 3 à 8, et **en ce que** l'une et/ou l'autre des électrodes comprend au moins un matériau comportant des fonctions hydroxyle et/ou un matériau carboné et/ou un additif carboné.

10. Système selon la revendication 9, **caractérisé en ce qu'**il est une batterie au lithium, une batterie au sodium, un supercondensateur à couche double électrique, un système hybride comprenant une électrode de batterie et une électrode de supercondensateur, ou bien encore une cellule électrochimique à flux continu.

## Patentansprüche

1. Verbindungen der folgenden Formeln (I-1) bis (I-3):

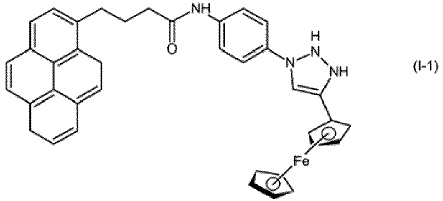

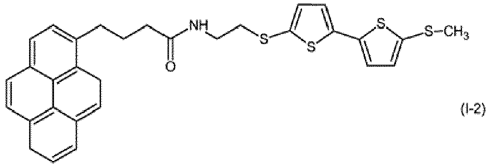

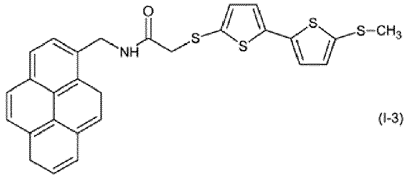

2. Verwendung mindestens einer Verbindung, ausgewählt aus den Verbindungen der Formel (I-1) bis (I-3) nach Anspruch 1 als Additiv in einer Elektrolytzusammensetzung eines elektrochemischen Systems mit faradayschen Elektroden und/oder mit Elektroden mit doppelter elektrischer Schicht oder pseudo-kapazitiven Elektroden, die durch Zirkulation von Ionen zwischen einer positiven Elektrode und einer negativen Elektrode funktionieren, die durch eine Elektrolytzusammensetzung getrennt sind.

3. Elektrolytzusammensetzung für ein elektrochemisches Systems mit faradayschen Elektroden und/oder mit Elektroden mit doppelter elektrischer Schicht oder pseudo-kapazitiven Elektroden, die durch Zirkulation von Ionen zwischen einer positiven Elektrode und einer negativen Elektrode funktionieren, die durch eine Elektrolytzusammensetzung getrennt sind, wobei die Elektrolytzusammensetzung **dadurch gekennzeichnet ist, dass** sie umfasst:
- mindestens eine ionische Verbindung,
- mindestens ein flüssiges Lösungsmittel,
- mindestens eine Verbindung, ausgewählt aus den Verbindungen der Formel (I-1) bis (I-3) nach Anspruch 1.

4. Zusammensetzung nach Anspruch 3, **dadurch gekennzeichnet, dass** die Menge Verbindung der Formel (I-1), (I-2) und/oder (I-3) innerhalb der Elektrolytzusammensetzung von 10⁻⁶ mol/l bis 2,10⁻¹ mol/l schwankt.

5. Zusammensetzung nach Anspruch 3 oder 4, **dadurch gekennzeichnet, dass** sie mindestens eine Verbindung der Formel (I-1) nach Anspruch 1 umfasst.

6. Zusammensetzung nach einem der Ansprüche 3 bis 5, **dadurch gekennzeichnet, dass** das elektrochemische System eine Batterie ist, die durch Zirkulation von Lithium- oder Natriumionen funktioniert und dass das Elektrolyt ein Lithium- oder Natriumsalz in Lösung in einem polaren aprotischen Lösungsmittel umfasst.

7. Zusammensetzung nach Anspruch 6, **dadurch gekennzeichnet, dass** das Elektrolyt ein Lithiumsalz umfasst, ausgewählt aus LiPF₆, LiAsF₆, LiClO₄, LiBF₄, LiC₄BO₈, Li(C₂F₅SO₂)₂N, Li[(C₂F₅)₃PF₃], LiCF₃SO₃, LiCH₃SO₃, LiN(SO₂CF₃)₂ und LiN(SO₂F)₂.

8. Zusammensetzung nach einem der Ansprüche 3 bis 5, **dadurch gekennzeichnet, dass** das elektrochemische System ein Superkondensator ist und dass die Elektrolytzusammensetzung ausgewählt ist aus:
- den Elektrolytzusammensetzungen, die ein Lithium- oder Natriumsalz in Lösung in einem polaren aprotischen Lösungsmittel und nach einem der Ansprüche 6 oder 7 umfassen;
- den wässrigen Lösungen mit neutralem pH wie die Lösungen von K₂SO₄ oder eines Salzes vom Typ Chlorat,
- den wässrigen Lösungen mit saurem pH wie die Schwefelsäurelösungen,
- den wässrigen Lösungen mit basischem pH wie die Kali- oder Natriumcarbonatlösungen.

9. Elektrochemisches System mit faradayschen Elektroden und/oder mit Elektroden mit doppelter elektrischer Schicht oder pseudo-kapazitiven Elektroden, die durch Zirkulation von Ionen zwischen einer positiven Elektrode und einer negativen Elektrode funktionieren, die durch eine Elektrolytzusammensetzung getrennt sind, **dadurch gekennzeichnet, dass** die Elektrolytzusammensetzung nach einem der Ansprüche 3 bis 8 ist und dass die eine oder die andere der Elektroden mindestens ein Material umfasst, das Hydroxylfunktionen aufweist und/oder ein karbonhaltiges Material und/oder ein karbonhaltiges Additiv.

10. System nach Anspruch 9, **dadurch gekennzeichnet, dass** es eine Lithiumbatterie, eine Natriumbatterie, einen Superkondensator mit doppelter elektrischer Schicht, ein Hybridsystem, das eine Batterieelektrode und eine Superkondensatorelektrode oder auch eine elektrochemische Zelle mit kontinuierlichem Fluss umfasst, ist.

## Claims

1. Compounds of the following formulae (I-1) to (I-3):

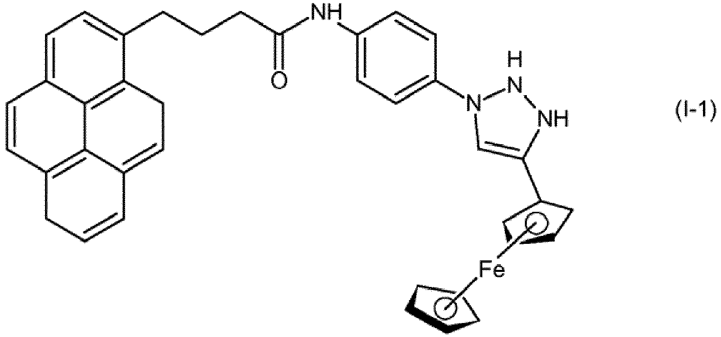

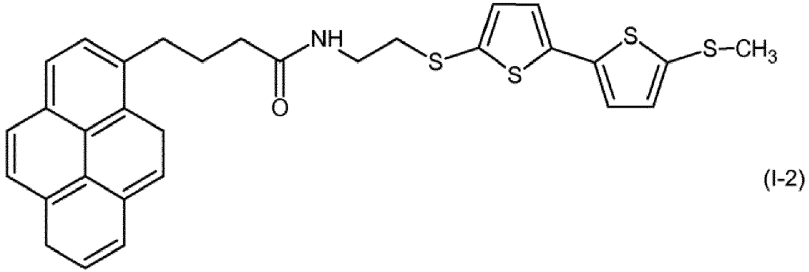

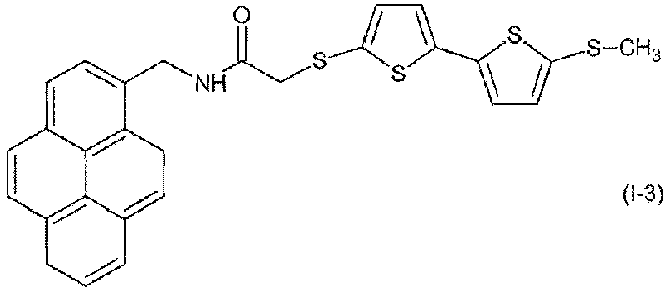

2. The use of at least one selected from among the compounds of formulae (I-1) to (I-3) as defined in the claim 1, as an additive in a composition of an electrolyte of an electrochemical system with Faradic electrodes and/or with electrodes with a double electric layer or pseudo-capacitive electrodes operating by circulation of ions between a positive electrode and a negative electrode separated by an electrolyte composition.

3. An electrolyte composition for an electrochemical system with Faradic electrodes and/or with electrodes with a double electric layer or pseudo-capacitive electrodes operating by circulation of ions between a positive electrode and a negative electrode separated by an electrolyte composition, said electrolyte composition being **characterized in that** it contains:
- at least one ionic compound,
- at least one liquid solvent,
- at least one compound selected from among the compounds of formulae (I-1) to (I-3), as defined in claim 1.

4. The composition according to claim 3, **characterized in that** the amount of compound of formula (I-1), (I-2) and/or (I-3) within the electrolyte composition varies from 10 mol/L to 2.10⁻¹ mol/L.

5. The composition according to claim 3 or 4, **characterized in that** it contains at least one compound of formula (I-1) as defined in claim 1.

6. The composition according to any of claims 3 to 5, **characterized in that** the electrochemical system is a battery operating by circulation of lithium or sodium ions and **in that** the electrolyte comprises a lithium or sodium salt in solution in a polar aprotic solvent.

7. The composition according to claim 6, **characterized in that** the electrolyte comprises a lithium salt selected from among LiPF₆, LiAsF₆, LiClO₄, LiBF₄, LiC₄BO₈, Li(C₂F₅SO₂)₂N, Li [(C₂F₅)₃PF₃], LiCF₃SO₃, LiCH₃SO₃, LiN(SO₂CF₃)₂ and LiN(SO₂F)₂.

8. The composition according to any of claims 3 to 5, **characterized in that** the electrochemical system is a supercapacitor and **in that** the electrolyte composition is selected from among:
- the electrolyte compositions comprising a lithium or sodium salt in solution in a polar aprotic solvent and/as defined in any of claims 6 or 7;
- the aqueous solutions of neutral pH such as solutions of K₂SO₄ or of a salt of the chlorate type;
- the aqueous solutions of acid pH, such as solutions of sulfuric acid;
- the aqueous solutions of basic pH, such as potash or soda solutions.

9. An electrochemical system with Faradic electrodes and/or with electrodes with a double electric layer or pseudo-capacitive electrodes operating by circulation of ions between a positive electrode and a negative electrode separated by an electrolyte composition, **characterized in that** the electrolyte composition is such as defined in any of claims 3 to 8, and **in that** both or either of the electrodes comprises at least one material including hydroxyl functions and/or a carbonaceous material and/or a carbonaceous additive.

10. The system according to claim 9, **characterized in that** it is a lithium battery, a sodium battery, a supercapacitor with a double electric layer, a hybrid system comprising a battery electrode and a supercapacitor electrode, or else further and electrochemical cell with continuous flow.
